Europäisches Patentamt

European Patent Office

Office européen des brevets

(19)

(11) Publication number: **0 265 066**
**A2**

(12) # EUROPEAN PATENT APPLICATION

(21) Application number: **87308079.0**

(22) Date of filing: **11.09.87**

(51) Int. Cl.⁴: **A61K 37/02** , **A61K 35/78**

(30) Priority: **11.09.86 JP 214955/86**
**16.12.86 JP 299326/86**
**15.04.87 JP 92683/87**

(43) Date of publication of application:
**27.04.88 Bulletin 88/17**

(84) Designated Contracting States:
**DE FR GB**

(71) Applicant: **Tamanoi Vinegar Corporation Limited**
**1-32, Kurumano-cho Nishi-1**
**Sakai-shi Osaka(JP)**

(72) Inventor: **Kubota, Terumasa**
**20-19, Aza-Hamagurizawa Ueta-cho**
**Toyohashi-shi Aichi(JP)**
Inventor: **Oshio, Tatsuichiro**
**18, Shugakuin Miyanowaki-cho Sakyo-ku**
**Kyoto-shi Kyoto(JP)**
Inventor: **Oki, Yuji**
**603, 9-33, Hishiya Nishi-6**
**Higashi Osaka-shi Osaka(JP)**
Inventor: **Haramaki, Yukari**
**6-5, Aioi-cho**
**Tsuruga-shi Fukui(JP)**
Inventor: **Saito, Fumio**
**21-16, Takaai-4 Higashi-Sumiyoshi-ku**
**Osaka-shi Osaka(JP)**
Inventor: **Yoshinaga, Atsunori**
**201, 2-22, Kaorigaoka-2**
**Sakai-shi Osaka(JP)**

(74) Representative: **Ablewhite, Alan James et al**
**MARKS & CLERK 57/60 Lincoln's Inn Fields**
**London WC2A 3LS(GB)**

(54) Antitumoral agent and process for preparing the same.

(57) This invention relates to an antitumoral agent of proteinic substance obtained by extraction and purification, from an unprocessed or processed seed having suffered thermal denaturation or having not, and a process for preparing the same.

The feature of the invention resides in subjecting said seed prior to extraction of proteinic substance or the proteinic substance extracted directly from said seed to the action of enzyme, and purifying the proteinic substance.

## Antitumoral agent and process for preparing the same

## BACKGROUND OF THE INVENTION

### Field of the Invention

This invention relates to an antitumoral agent of proteinic substance obtained by extraction and purification, from an unprocessed or processed seed having suffered thermal denaturation or having not, and a process for preparing the same. In this specification the term "proteinic substance" is applied to protein and protein-containing substance, for example, comprising polypeptide of molecular weight 500-5000, and the term "seeds" covers seeds, fruits, berries and nuts.

### Description of the Prior Art

So far have been known processes for preparing antitumoral agents or substances, for example, comprising purifying antitumoral substances which produce varying types of cells or microorganisms, comprising recovering antitumoral substances from processed cereals with the surface layers heat-and pressure-treated (Japanese Laid-open Patent No. 13971/1978), comprising extracting furze seeds with hot water (Japanese Laid-open Patent No. 154508/1979).

Besides a technique comprising fermenting cereals under the work of Rhizops oryzae strain M24, and recovering antitumoral agent from the fermentation liquor has been disclosed (Japanese Published Patent No. 41551/1986).

Hitherto residues coming out of the process for producing vinegar have been used as fodder.

## SUMMARY OF THE INVENTION

The above-mentioned process of prior art where cells is used has disadvantages of difficult mass cultivation and expensive culture medium. From the viewpoint of the aspect of safety, those derived from microorganisms are required to be highly purified. The method including extraction from cereals has difficulties in industrial application: for one thing, the agents may be non-proteinic polysaccharides and so a large amount of energy is required for extraction of them, and for another thing, pure culture of the concerned strain is inevitable. In the last method comprising extracting from furze, there is suspectability of natural effective components to be denatured or damaged.

The present invention has features and advantages over those method stated above: One of them resides in purifying proteinic substances from unprocessed or processed seeds having not been thermally denatured, and thus they have great antitumoral activities in relatively small doses.

Another preferred feature of the invention resides in preparing from processed or unprocessed seeds having not been denatured in the following either procedure: Firstly, such a seed (one part) is ground if necessary. To it, an aqueous medium (1-10 parts) is added, followed by alcohol or acetic acid (0.1-10%) for antisepsis as desired, enzyme preparation (0.01-5% based on the weight of the raw material) consisting of protease (endopeptidase) as a main component, glucoamylase, pectinase, and others. The resulting mixture is subjected to extraction (3-50°C; 1 to 15 days). Secondly, to the seed (one part), an aqueous medium (1-10 parts) is added, and proteins are extracted. To the extract, an enzyme preparation (0.01-5%) consisting essentially of protease (endopeptidase) is added, and then incubated at 3-50°C for 1 to 10 days. When amino acid equivalent (referred to as A.E. hereinafter) which is the rate of amino acid nitrogen to total nitrogen, has reached 10-70%, the reaction solution is subjected to centrifugation or filtration to remove the solids. To the supernatant or filtrate, ammonium sulfate is added to 100% saturation, and the resulting precipitation fractions are in turn collected together by centrifugation, dissolved in distilled water, desalted through dialysis, and freeze-dried. Thus an antitumoral substance can be obtained. The solid-free super natant or filtrate may be purified an alternative known technique, such as ion exchange, or gel filtration, if required. A.E. is within the range of 10 to 70% in above-described procedure because it has been demonstrated that the efficiency rises with increasing A.E., but gradually lowers as A.E. approaches to 70% and that poor efficiencies result when A.E. is out of this range.

Antitumoral activity has been examined in vitro as follows: Balb/c3T3 cell (mouse embryo fibroblast) was employed as a normal cell, and the $SV_{40}$ virus-transformed cell mKSA•4AC from normal ones as a tumor cell. The fifth cultivation day cells were suspended in a 10% fetal bovine serum containing MEM culture medium, and spread in Petri's dish (inner diameter: 6 centimeter) to make a population of $1 \times 10^5$, and a sample preparation (1 milliliter) which was prepared by dissolving the sample in the same culture medium, filtering and sterilize, is added to make totally 4 milliliter of the culture medium, and then incubated at 37°C in an atmosphere of 5% $CO_2$. On the fifth day of cultivation, Giemsa's staining was made and measurement with a cytometer (Olympus, Jap) to obtain growth index against control (only culture medium added). The yielded results are summarized in Table 1.

Table 1 clearly reveals that substances according to the present invention have a marked growth-inhibitory effect on tumor cells and no toxicity to normal cells.

Table 1

| Example | Concentration ($\mu$ g/ml) | Growth rate(%) | |
|---|---|---|---|
| | | Tumor cell | Normal cell |
| 1 | 25 | 2 | 95 |
| | 50 | 9 | 93 |
| 2 | 25 | 0 | 102 |
| | 50 | 0 | 93 |
| 3 | 25 | 2 | 97 |
| | 50 | 10 | 98 |
| 4 | 25 | 15 | 102 |
| | 50 | 53 | 97 |
| 5 | 25 | 19 | 95 |
| | 50 | 63 | 97 |

Further in vivo study on the antitumoral activity was done as follows: groups of 6 female (aged 8 weeks) mice of ICR strain, weighing 28± 1 gram, were employed. Sarcoma-180 ($2 \times 10^5$) was implanted in the abdominal cavity. For 5 days beginning with the day, a suspension of a sample in phosphate buffer saline (PBS) was intraperitoneally administered daily. On the 60th, the number of survivors was counted and increase in lifespan (ILS) was calculated to evaluate the antitumoral activity. As control, animals given PBS only were employed. The yielded results are shown in Table 2.

3

Table 2

| Example | Dosage mg/mouse/day | MSD(day) | ILS(%) | No.of survivors |
|---------|---------------------|----------|--------|-----------------|
| Control |                     | 9.5      | 0      | 0               |
| 1       | 0.5                 | 23       | 142    | 4               |
| 2       | 0.5                 | 27       | 184    | 5               |
| 3       | 0.5                 | 20       | 111    | 4               |
| 4       | 1.0                 | 22       | 132    | 3               |
| 5       | 1.0                 | 18       | 89     | 3               |

MSD: Median survival day (for died animals)
ILS: Increase in lifespan (for died animals)
ILS (%) = {(T - C)/C} x 100
where C is MSD of control group and T is MSD of treated group.

From Table 2, it has become apparent that likewise in vivo substance according to the present invention have a reemarkable antitumoral activity.

Another feature of the invention in which starch is removed before extraction of proteins will be described in the following:

This feature resides in comprising subjecting a seed having been not thermal denaturation to mechanical, enzymatic or biological treatment, or various combinations of them so as to remove starch, extracting proteinic substance with an aqueous medium containing enzymes added thereto and purifying the proteinic substance from the resultant extract solution.

The above-mentioned mechanical treatment can be accomplished, for example, by destroying husks of cereals e.g. by grinding, and then removing amylaceous substances, e.g. by sieving. The afore-said enzymatic treatment implies method for removing amylaceous substances with enzymes alone or in combination. The above-mentioned biological treatment is, for example, method for which the action of molds are utilized. For efficient removal of amylaceous substances, the above-listed three methods should be applied alone or in combination, taking properties of cereals into consideration. There are various practical applications: for example, heat-untreated cereals are immersed in water, wet-ground and sieved to obtain amylaceous-free substance; as an alternative procedure, the raw material (one part) is in turn ground, suspended in water (10 parts), and subjected to the action of enzyme (0.01 to 5% based on the weight of the raw material) consisting essentially of glucoamylase, followed by to efficient removal of amylaceous substances. Then, after addition of yeast for preventing from contamination with infectious microbes, the reaction mash is incubated at 20-35°C for 2 to 6 days, thus alcohol fermentation being accomplished, to remove amylaceous substances at removal rates of 80 to 99%. The fermentation liquor is subjected to centrifugation or filtration to obtain a starch-free product. To the product (one part), aqueous medium (0.1 to 20 parts) alone or containing enzyme (0.01-5%) consisting mainly of protease (endopeptidase) added thereto, and in cubated at 3 to 50°C for 1 to 15 days to extract proteinic substances. The extract is centrifuged to remove solids, and saturated with ammonium sulfate up to 100%. The resulting precipitate fractions are subjected to dialysis to desalt, and freeze-drying. Thus an antitumoral substance is obtained.

The description of the products according to the invention (Examples 6 through 9) will be given the following:

4

Table 3

| Example | Concentration ( μ g/ml ) | Growth rate (%) | |
| --- | --- | --- | --- |
| | | Tumor cell | Normal cell |
| 6 | 25 | 2 | 97 |
| | 50 | 7 | 97 |
| 7 | 25 | 2 | 93 |
| | 50 | 10 | 89 |
| 8 | 25 | 13 | 97 |
| | 50 | 32 | 95 |
| 9 | 25 | 15 | 102 |
| | 50 | 33 | 92 |

The results of antitumoral activity studied in vitro in the same way as above-stated are summarized in Table 3.

Table 3 demonstrates that the products according to the invention have a marked growth-inhibitory effect on tumor cells and no toxicity to normal cells.

The results of antitumoral activity evaluated in vivo in the same way as above-stated are shown in Table 4.

Table 4

| Example | Dosage mg/mouse/day | MSD(day) | ILS(%) | No.of survivors |
| --- | --- | --- | --- | --- |
| Control | | 10 | 0 | 0 |
| 6 | 0.5 | 25 | 150 | 5 |
| 7 | 0.5 | 28 | 180 | 3 |
| 8 | 1.0 | 22 | 120 | 3 |
| 9 | 1.0 | 23 | 130 | 3 |

From Table 4, it has become apparent that likewise in vivo, the products according to the invention have marked antitumoral activities.

A further feature according to the invention is as follows: a heat-untreated seed having suffered none of conversion of starch into α-form, denaturation of protein, and other thermal decomposition of other components, for instance, by heating, is used as raw material. The seed (one part) is ground and mixed with water (2-7 parts). To the mixture are added a group of glucoamylase (2-20 units), protease (1-10 units), α-amylase (5-50 units), pectinase (0.1-0.5 units), cellulase (0.01-0.1 units) and lipase (1-60 units), unit values in parentheses representing enzymatic activity per gram of the raw material, and self-produced or commercially-available cultured yeast, and incubated at 20-35°C for 2-7 days during which saccharification of starch, dissolution of components, and alcohol fermentation proceed. Then acetic acid bacteria are inoculated in the fermented mash and reincubated at 25-35°C for 1 to 14 days. After completion of this fermentation are made filtration or centrifugation for removing solids, followed by diatomaceous earth and membrane refiltrations of the filtrate or supernatant for removal of insoluble components and bacteria. The resulting clear liquor is saturated with ammonium sulfate added thereto at degrees of 50 to 100%. Fractions of precipitation are dissolved with distilled water. The clear solution is either dialysed and freeze-dried, or ultra-filtered with a membrane impermeable to molecules having MW of 5000 or more, followed by freeze-drying of the residue on the membrane. In this way, an antitumoral agent can be obtained.

Activities and potencies of enzymes for use in the invention were determined as follows:

Glucoamylase:

To reducing sugar by subjecting 0.5% starch solution to the action of this enzyme is added Fehling's solution, and heated with the result of precipitation of cuprous oxide. Surplus copper is titrated under sulfuric acid-acidified condition with sodium thiosulfate in the presence of potassium iodide.

Potency: One unit = Enzymatic activity that produces 10 milligram glucose at 30±1°C and pH 4.5 in 30 minutes.

Protease:

1.5% milk casein solution is mixed with enzyme and incubated. The thus-obtained trichloroacetic acid-soluble fraction is color-produced with alkaline Folin's reagent and then the absorbance is measured at 660nm.

Potency: One unit = Enzymatic acitivity that produces amino acid correspond to 1 milligram of tyrosine at pH 4.5 and 30±1°C in 60 minutes.

α-amylase:

0.5% starch solution is subjected to the action of enzyme, and the reducing rate of iodine-starch reaction color-development is determined by colorimetry (660nm).

Potency: One unit = Enzymatic activity that brings about 1%-reduction of color-development of pH 4.5 and 30±1°C in 1 minute.

Pectinase:

0.5% pectin solution is exposed to the action of enzyme, and the reducing rate of the viscosity is determined with a viscometer.

Potency: One unit = Enzymatic activity that reduces 50% viscosity at pH 4.5 and 30±1°C in 1 minute.

Cellulase:

0.625% CMC-Na solution is subjected to the action of enzyme, and the produced reducing glucose is determined in the same way under "Glucoamylase".

Potency: One unit = Enzymatic acitivity that produces 10 milligram of glucose at pH 4.5 and 30±1°C in 30 minutes.

Lipase:

20% olive oil emulsion is exposed to the action of enzyme, and the produced fatty acids are titrated with sodium hydroxide.

Potency: One unit = Enzymatic activity that produces 1 μ mol of fatty acid at pH 4.5 and 30±1°C in 1 minute.

Table 5

| Example | Raw material | Purifi-cation | Protein (%) | Sugar (%) |
|---------|--------------|---------------|-------------|-----------|
| 10 | Pearl barley | Salting out | 67.3 | 29.0 |
| 11 | Pearl barley | Ultra-filtraion | 77.1 | 18.9 |
| 12 | Unpolished rice | Salting out | 65.1 | 30.4 |
| 13 | Corn | Salting out | 57.3 | 35.6 |

Further the results of analysis of effective components according to the invention obtained in Examples 10 through 13 are summarized in Table 5.

From Table 5, it becomes apparent that the substances according to the invention are proteinic. The analyses were conducted as follows:

Protein: Lowry method (C. D. Stauffer, Analytical Biochemistry 69, 646, 1975)

Sugar: Phenol-sulfuric acid method (M.Dubois, K.A.Gilles, J.K.Hamilton, P.A.Revers, and F.Smith: Analytical Chemistry 28,350,1956)

In vitro antitumoral activity has been studied in the following way: mKSA•4AC which is the $SV_{40}$ virus-transformed Balb/c3T3 of mouse embryo fibroblast was employed as a tumor cell. Eight Petri's dishes (inner diameter: 6 centimeter) were used every sample. The fifth cultivation day cells were suspended in a 10% fetal bovine serum-containing MEM culture medium, and spread in a Petri's dish to make a population of $1 \times 10^5$. To it, 1 milliliter of a sample preparation dissolved in the same culture medium was added, and then incubated at 37°C in an atmosphere of 5% $CO_2$ for 5 days. The number of cells was counted, and the addition of only culture medium was used as control. The yielded results are summarized in Table 6.

Table 6 clearly demonstrates that substances according to the invention have a marked inhibitory effect on growth of cells, and additionally cooking results in loss of the activities.

Table 6

| Example | Raw material | Purification | Concentration (mg/ml) | *DETC (%) |
|---|---|---|---|---|
| 10 | Pearl barley | Salting out | 0.4<br>0.8 | 59.9<br>93.3 |
| 11 | Pearl barley | Ultrafiltraion | 0.5<br>1.0 | 43.2<br>90.3 |
| 12 | Unpolished rice | Salting out | 1.0<br>2.0 | 50.1<br>89.3 |
| 13 | Corn | Salting out | 1.0<br>2.0 | 20.5<br>59.8 |
| | Pearl barley | ※ Heat-treatment | 0.5<br>1.0 | 7.2<br>3.8 |

*DETC: Destructive effect on tumor cell (mKSA)
Destructive effect: (%) = {(C - T)/C} x 100
where C is the number of cells in control group, and T is the number of cells in sample-treated Group.
※ The procedure comprises cooking raw material (pearl barley) to cause starch to liquefy and saccharize, and subsequently in the same way as the process according to the invention, performing ammonium sulphate-fractional precipitation, the resulting precipitates at 50 to 100% saturations beeing dialysed and freeze-dried.

A white powder was obtained at a yield of 4.1 gram per raw material 1 kilogram.
Further in vivo study on the antitumoral activity was done as described above, and the yielded results are shown in Table 7.

Table 7

| Example | Raw material | Sample Purification | Dosage(mg/mouse/day) | MSD (day) | ILS (%) | No.of Survs |
|---|---|---|---|---|---|---|
| Control | | | | 9.7 | 0 | 0 |
| 10 | Pearl barley | Salting out | 1mg | 25 | 158 | 5 |
| 11 | Pearl barley | Ultrafiltration | 2mg | 32 | 230 | 4 |
| 12 | Unpolished rice | Salting out | 2mg | 21 | 116 | 4 |
| 13 | Corn | Salting out | 2mg | 19 | 96 | 5 |
| | Pearl barley | ※Heat treatment | 2mg | 18 | 86 | 0 |

※ The same procedure as that in Table 6.

It becomes apparent from Table 7 that likewise in vivo, the substances according to the invention have marked antitumoral activities.

Besides the results of antitumoral activity yielded in Examples 14 through 16 were evaluated in the same way as described above and summarized in Table 8.

Table 8 reveals that the substances according to the invention have extremely-high antitumoral activities, and in particular the group of 10 milligram dose-treated mice gave the result of complete healing, and this suggests these substances are promising to be epoch-making carcinostatic agents.

Further other Examples 17 through 20 according to the invention are outlined in the following: seeds are used as raw material. A seed (one part) is ground and mixed with water(2-20 parts). To the mixture are added a group of glucoamylase (0.01-2%), and, if necessary, α-amylase, protease, pectinase and cellulase (0.01-0.2%). For higher

Table 8

| Example | Dosage mg/mouse/day | MSD(day) | ILS(%) | No.of survivors |
|---------|--------------------|----------|--------|-----------------|
| Control |  | 9.7 | 0 | 0 |
| 14 | 5 | 20 | 106 | 5 |
|  | 10 | 60< | 519< | 6 |
| 15 | 5 | 17.5 | 80 | 4 |
|  | 10 | 60< | 519< | 6 |
| 16 | 5 | 18 | 86 | 3 |
|  | 10 | 60< | 519< | 6 |

efficient dissolution of amylaceous substances and for prevention of contamination with infectious microbes, yeast is added to cause alcohol fermentation. Then dissolution was conducted at 15-60°C for 4-20 days. Then filtration or centrifugation was made to remove solids. The filtrate or supernatant was saturated with ammonium sulfate added thereto at degrees of 50 to 100%. The fractions of precipitation are dissolved with distilled water. The clear solution was either dialysed and freeze-dried, or ultrafiltrated with a membrane of a fractional MW of 10000 to desalt, followed by concentration and freeze-drying. In this way, an antitumoral agent can be obtained. If required, additional purification may done by known techniques such as ion exchange, gel filtration, etc.

The above-stated seeds includes such as pearl barley, unpolished rice, and corn.

The products according to the invention will be described hereinafter:

Table 9 gives analytical compositions of cereals used.

Table 9

| Example | Raw material | Purifi-cation | Protein (%) | Sugar (%) |
|---|---|---|---|---|
| 17 | Pearl barley | Salting out | 74.7 | 21.3 |
| 18 | Pearl barley | Ultra-filtraion | 68.9 | 25.2 |
| 19 | Unpolished rice | Salting out | 61.3 | 32.5 |
| 20 | Corn | Salting out | 73.2 | 20.5 |

Protein was determined by the Lowry method and sugar by the phenol sulfuric acid method.

Table 9 shows that the products according to the inven tion are proteinic.

Study on antitumoral activities in vitro was made in the same way as stated above, and the yielded results are summarized in Table 10.

Table 10

| Exam-ple | Raw mate-ial | Purifi-cation | Concent-ration ($\mu$g/ml) | Growth rate(%) | |
|---|---|---|---|---|---|
| | | | | Tumor cell | Normal cell |
| 14 | Pearl bar-ley | Salting out | 50<br>100 | 17<br>0 | 93<br>78 |
| 15 | Pearl bar-ley | Ultra-filtraion | 100<br>200 | 51<br>17 | 89<br>68 |
| 16 | Unpolished rice | Salting out | 100<br>200 | 63<br>42 | 87<br>72 |
| 17 | Corn | Salting out | 100<br>200 | 59<br>35 | 83<br>65 |

Table 10 demonstrates that the substances according to the invention have marked growth-inhibitory effect on tumor cells and low toxicity to normal cells.

Besides the antitumoral activities in vivo were studied in the same way as stated above, and yielded results are shown in Table 11.

From Table 11 it becomes apparent that the substances according to the invention have also in vivo a marked antitumoral activities.

Table 11

| Exa-mple | Raw material | Purification | Dosage(mg/mouse/day) | MSD (day) | ILS (%) | No.of Survs |
|---|---|---|---|---|---|---|
| Con-trol | | | | 9.5 | 0 | 0 |
| 17 | Pearl berlay | Salting out | 0.5 | 20 | 111 | 4 |
| 18 | Pearl berlay | Ulfrafiltrn. | 1.0 | 22 | 132 | 3 |
| 19 | Unpolished rice | Salting out | 2.0 | 25 | 163 | 3 |
| 20 | Corn | Salting out | 2.0 | 18 | 89 | 3 |

Microorgansims for use in the present invention, yeasts and acetic acid bacteria, have been used for sakes and vinegars from olden times in Japan,. and safeties of the fermentation products have been established historically. It is noteworthy that major components of the fermentation products, i.e. ethanol and acetic acid, have germicidal power, and thus the fermentations are much easy to be controlled with less equipment as compared with cell culture.

In the following procedures of the invention where raw material is to suffer thermal denaturation will be described: an unprocessed or processed seed (one part) is ground, if necessary, and mixed with water (2.0-7.0 parts) added thereto. The mixture is adjusted to pH 4.5, and remixed with commercially-available glucoamylase (0.01-1% based on the weight of the raw material), which is preferred in efficiency when alkali-treated to destroy the protease activity before use, and yeast (0.05-1% based on the weight of the raw material), and then incubated at 25-35°C over 2 to 7 days for alcohol fermentation without cooking. The fermentation liquor is denatured as predetermined, and then filtered or centrifuged to separate into solids (residue) and separated liquor (vinegar mash). The latter is subjected to acetic acid fermentation to produce vinegar. The residue is further processed in the following procedure (A) to obtain an antitumoral substance.

Procedure A: To the residue (one part) is added water (1-10 parts) and heat-sterilized at 60 to 120°C. The acetic acid (0.1-10%) for antisepsis, followed by commercially available protease, glucoamylase, pectinase and cellulase (0.01-5% base on the weight of the residue), are added and incubated at 3-50°C for 2 to 20 days. When A.E. has reached 10 to 70%, especially preferably 30 to 50%, solids are removed by centrifugation or filtration. To the supernatant or filtrate is added ammonium sulfate to 100% saturation, and the fractions of precipitation are collected together by centrifugation. The resultant is dissolved with distilled water, dialysed for desalting and freeze-dried. Thus an antitumoral substance can be obtained.

An alternative procedure will be described under: a seed (one part) is ground and mixed with water(2.0-7.0 parts) added thereto. To the mixture is added a commercially-available α-amylase (0.1-0.5% based on the weight of the raw material), and then incubated at 60°C over 30 minutes for liquefaction, followed by cooking at 120°C and 2 atmospheric pressure for 5 minutes, and then adjustment to pH 5.0. Further a commercially-available glucoamylase (0.1 to 1% based on the weight of the raw material) is added, and incubated at 55°C over 16 hours for saccharification.

The saccharified mash is either filtered or centrifuged to separate into solid residue (a) and sugar solution, or adjusted to pH 4.5 followed by addition of yeast (0.05-1% based on the weight of the raw material), and incubated at 25-35°C for 2 to 7 days, thus fermentation being accomplished. After a predetermined denaturation is made filtration or centrifugation for separation into solid residue (b) and vinegar mash.

The obtained sugar solution is used as it is or for alcohol fermentation. With the vinegar mash, acetic acid fermentation is made to produce vinegar. On the other hand, residue (a) separated from the sugar solution and residue (b) separated from vinegar mash can be used to obtain antitumoral substance in accordance with procedure A. In this case, heat-sterilization may be omitted.

In an alternative procedure where up to saccharification is the same as above-stated, then the sugar solution is adjusted to pH 4.5, and yeast (0.05-1%) and commercially-available glucoamylase, protease, pectinase and cellulase (0.01-5%) are added and incubated 25-35°C over 2 to 15 days for fermentation and then extraction. When the A.E. value of the extract reaches 10 to 70%, particularly 30-50% being better in efficiency, a predetermined denaturation is conducted. Then filtration or centrifugation is made to remove solids, and the filtrate or supernatant is processed in the same way as procedure A. Thus an substance can be obtained.

The antitumoral activities in vitro were studied as follows: Balb/c3T3 cell (mouse embryo fibroblast) was employed as a normal cell, and the $SV_{40}$ virus-transformed cell mKSA•4AC from normal ones as a tumor cell. The fifth cultivation day cells are suspended in a 10% fetal bovine serum containing MEM culture medium, and spread over a Petri's dish (inner diameter: 6 centimeter) to make a population of $1 \times 10^5$, and 1 milliliter of a sample preparation which is prepared by dissolving the sample in the same culture medium, followed by filtration and sterilization, is added to make totally 4 milliliter of the culture medium, and then incubated at 37°C in an atmosphere of 5% $CO_2$. On the fifth day of cultivation, Giemsa's staining was made and measurement with a cytometer (Olympus, Jap) to obtain growth index against control (only culture medium added). The yielded results are summarized in Table 2.

Table 12

| Example | Concentration $\left(\mu\ g/ml\right)$ | Growth rate $\left(\%\right)$ | |
|---|---|---|---|
| | | Tumor cell | Normal cell |
| 21 | 20 | 8 | 103 |
| | 40 | 0 | 95 |
| 22 | 20 | 23 | 97 |
| | 40 | 0 | 95 |
| 23 | 20 | 43 | 101 |
| | 40 | 7 | 103 |
| 24 | 20 | 10 | 98 |
| | 40 | 0 | 97 |
| 25 | 80 | 45 | 95 |
| | 160 | 14 | 98 |

Table 12 clearly reveals that substances according to the invention has a marked growth-inhibitory effect on tumor cells and no toxicity to normal cells.

Further in vivo study on the antitumoral activities were done as follows: groups of 6 female (age: 8 weeks) mice of ICR strain, weighing 28± 1 gram, were employed. Sarcoma-180 ($2 \times 10^5$)was transplanted in the abdominal cavity. For the following 5 days, a suspension of a sample in phosphate buffer saline (PBS) was intraperitoneally injected daily. On the 60th from the day of implantation, the number of survivors was counted and ILS was calculated to evaluate the antitumoral activities. As control, animals given PBS only were employed. The yielded results are shown in Table 13.

0 265 066

Table 13

| Example | Dosage mg/mouse/day | MSD(day) | ILS(%) | No.of survivors |
|---------|---------------------|----------|--------|-----------------|
| Control | - | 9.7 | 0 | 0 |
| 21 | 0.5 | 26 | 168 | 3 |
| 22 | 0.5 | 28.5 | 194 | 2 |
| 23 | 0.5 | 23 | 137 | 2 |
| 24 | 0.5 | 22 | 127 | 2 |
| 25 | 3.0 | 24 | 147 | 1 |

MSD: Median survival days (for died animals)
ILS: Increase in lifespan (for died animals)
ILS = ((T - C)/C) x 100

From Table 13, it has become apparent that likewise in vivo it has a remarkable antitumoral activities.

The proteinic substances according to the invention have proved to have antitumoral or carcinostatic activities. The present invention permits easy extraction of the proteinic substances from natural or processed seeds. In this specification the term "enzyme" is applied to enzyme and fermentation broth of microorganism which can produce protease.

## DESCRIPTION OF THE PREFERRED EMBODIMENTS

Example 1

Pearl barley (1 kilogram) was ground, and mixed with water (2.5 liters), ethanol (125 milliliter), and combined enzyme consisting of commercially-available protease (endopeptidase, 2 gram) and pectinase (2 gram) added thereto. For extraction, the mixture was incubated at 30°C for 6 days. When A.E. value has been 36%, centrifugation (4000×g) was made for 5 minutes to remove solids, and the supernatant was filtered with diatomaceous earth and then membrane (0.45μ m). To the resulting clear liquor, ammonium sulfate was added, and the 100% saturation fraction of precipitation was in turn dissolved with distilled water, dialysed and freeze-dried. Thus an antitumoral substance (1.1 gram) of yellow powder was obtained.

Example 2

With uncooked pearl barley (1 kilogram), alcohol fermentation was carried out. The resultant residue (water content: 60%, 1 kilogram) was mixed with water (5 liters), acetic acid(200 milliliters) and commercially-available protease (endopeptidase, 2 gram) added thereto. For extraction, the mixture was incubated at 35°C for 5 days. When A.E. value has been 37%, filtration was conducted to remove solids, and the filtrate was refiltered with a membrane (0.45μ m). To the resulting clear liquor, ammonium sulfate was added, and the 100% saturation fraction of precipitation was in turn dissolved with distilled water, dialysed and freeze-dried. Thus an antitumoral substance (0.9 gram) of brown powder was obtained.

Example 3

Pearl barley (1 kilogram) was ground, and mixed with phosphate buffer solution (50mM, pH 7.0) and ethanol(150 milliliter) added thereto. For extraction, the mixture was incubated at 30°C for 2 days. The solids was removed by filtration. To the filtrate, commercially-available protease (endopeptidase, 2 gram) and pectinase (1 gram) were added, and for conversion, incubated at 40°C for 2 days. When A.E. value has been 38%, filtration was done through a membrane (0.45μ m). To the filtrate, ammonium sulfate was added, and the 100% saturation fraction of precipitation was in turn dissolved with distilled water, dialysed and freeze-dried. Thus an antitumoral substance (1.2 gram) of yellow powder was obtained.

Example 4

Corn (1 kilogram) was ground, and mixed with water (2.5 liters), ethanol (125 milliliter), and combined enzyme consisting of commercially-available protease (endopeptidase, 2 gram) and pectinase (2 gram) added thereto. For extrac tion, the mixture was incubated at 30°C for 5 days. When A.E. value has been 43%, centrifugation (4000×g) was made for 5 minutes to remove solids, and the supernatant was filtered with diatomaceous earth and then a membrane (0.45μ m). To the resulting clear liquor, ammonium sulfate was added and the 100% saturation fraction of precipitation was in turn dissolved with distilled water, dialysed and freeze-dried. Thus an antitumoral substance (0.9 gram) of light yellow powder was obtained.

Example 5

Unpolished rice (1 kilogram) was ground, and mixed with water (2.5 liters), ethanol (125 milliliter), and combined enzyme consisting of commercially-available protease (endopeptidase, 2 gram) and pectinase (2 gram) added thereto. For extraction, the mixture was incubated at 30°C for 5 days. When A.E. value, has been 31%, centrifugation (4000×g) was made for 5 minutes to remove solids, and the supernatant was filtered with diatomaceous earth and then a membrane (0.45μ m). To the resulting clear liquor, ammonium sulfate was added, and the 100% saturation fraction of precipitation was in turn dissolved with distilled water, dialysed and freeze-dried. Thus an antitumoral substance (0.5 gram) of light yellow powder was obtained.

Example 6

Pearl barley (1 kilogram) was ground, and mixed with water (2.7 liters), and commercially-available glucoamylase (2 gram) and bakers yeast (3 gram) added thereto. For alcohol fermentation, the mixture was incubated at 30°C for 4 days. The fermented mash was centrifuged at 4000×g for 5 minutes to obtain starch-free precipitate. To the product, phosphate buffer solution (50mM, pH 7.0; 5 liters) was added, and extraction was made with stirring. Recentrifugation (5000×g) was carried out for 5 minutes to remove solids. To the extract, ammonium sulfate was added, and the 100% saturation fraction of precipitation was in turn dissolved with distilled water, dialysed and freeze-dried. Thus an antitumoral substance (1.2 gram) of yellowish brown powder was obtained.

Example 7

Pearl barley (1 kilogram) was washed with water, and immersed in water (5 liters) for one day. After swishing water off, water (2 liters) was added, wet-ground over 5 minutes, and sieved through 100 meshes to remove starch. To the starch-free residue were added water (3 liters), acetic acid (120 milliliter) and commercially-available protease (1 gram) and pectinase (1 gram), and for extraction, the mixture was incubated at 30°C for 7 days. Centrifugation (4000×g, 5 minutes) was made to remove solids. To the ex tract ammonium sulfate was added, and the 100% saturation fraction of precipitation was in turn dissolved with distilled water, dialysed and freeze-dried. Thus an antitumoral substance (0.9 gram) of yellowish brown powder was obtained.

14

Example 8

Corn (1 kgram) was ground, and mixed with water (2.7 liters), and commercially-available glucoamylase (2 gram) and bakers yeast (3 gram) added thereto. For alcohol fermentation, the mixture was incubated at 30°C for 4 days. The fermented mash centrifuged at 5000$^\times$g for 5 minutes to obtain starch-free precipitate. To the starch-free product were added water (5 liters), acetic acid (200 milliliter) and commercially-available protease (1 gram) and pectinase (0.5 gram), and extracted at 30°C for 6 days. Recentrifugation (5000$^\times$g) was carried out for 5 minutes to remove solids. To the extract, ammonium sulfate was added, and the 100% saturation fraction of precipitation was in turn dissolved with distilled water, dialysed and freeze-dried. Thus an antitumoral substance (0.8 gram) of light yellow powder was obtained.

Example 9

Unpolished rice (1 kilogram) ground, and mixed with water (2.7 liters), and commercially-available glucoamylase (2 gram) and bakers yeast (3 gram) added thereto. For al cohol fermentation, the mixture was incubated at 30°C for 4 days. The fermented mash was centrifuged at 5000$^\times$g for 5 minutes to obtain starch-free precipitate. To the starch-free product were added water (5 liters), acetic acid (200 milliliter) and commercially-available protease (1 gram) and pectinase (0.5 gram), and extracted at 30°C for 10 days. Recentrifugation (5000$^\times$g) was carried out for 5 minutes to remove solids. To the extract, ammonium sulfate was added, and the 100% saturation fraction of precipitation was in turn dissolved with distilled water, dialysed and freeze-dried. Thus an antitumoral substance (0.7 gram) of light yellow powder was obtained.

Example 10

Pearl barley (1k gram) was ground, and mixed with water (2.7 liters), and commercially-available glucoamylase (2 gram), protease (1 gram) and bakers yeast (3 gram) added thereto. For fermentation, the mixture was incubated at 28-30°C for 4 days. To the fermented mash, acetic acid bacteria culture was inoculated and incubated at 28-30°C over 6 days for fermentation. Upon the last fermentation has completed, the fermentation liquor was centrifuged at 4000$^\times$g for 10 minutes to remove solids. The supernatant was filtered with a membrane, and ammonium sulfate was added. The precipitates from the fractions of 50-100% saturation were in turn dissolved with distilled water, dialysed and freeze-dried. Thus an antitumoral substrate (7.3 gram) of white powder was obtained.

Example 11

Pearl barley (1 kilogram) was ground, and mixed with water (3.0 liters), and commercially-available glucoamylase (2 gram), pectinase (0.5 gram) and previously cultured fresh yeast (3 gram) added thereto. For fermentation, the mixture was incubated at 28-30°C for 6 days. To the fermented mash, acetic acid bacteria culture was inoculated and incubated over 10 days for fermentation. When the last fermentation has completed, filtration was made to remove solids. The filtrate was subjected to ultrafiltration through a membrane permeable to molecules of MW: less than 5000, and the residue on the membrane was freeze-dried. Thus an antitumoral substance (15.3 gram) of light yellow powder was obtained.

Example 12

Unpolished rice (1 kilogram) was ground, and mixed with water (2.7 liters), and commercially-available glucoamylase (2 gram), cellulase (1 gram) and bakers yeast (3 gram) added thereto. For fermentation, the mixture was incubated at 28-30°C for 5 days. To the fermented mash, acetic acid bacteria culture was inoculated and incubated at 28-32°C over 6 days for fermentation. When the last fermentation has completed, the fermentaion liquor was centrifuged at 4000$^\times$g for 10 minutes to remove solids. The supernatant was filtered with a membrane, and ammonium sulfate was added. The precipitates from the fractions of 50-100% saturation were in turn dissolved with distilled water, dialysed and freeze-dried. Thus an antitumoral substance (3.9 gram) of light yellow powder was obtained.

Example 13

Corn (1 kilogram) was ground, and mixed with water (2.7 liters), and commercially-available glucoamylase (2 gram), and bakers yeast (3 gram) added thereto. For fermentation, the mixture was incubated at 28-30°C for 4 days. To the fermented mash, acetic acid bacteria culture was inoculated and incubated at 28-32°C over 10 days for fermentation. When the last fermentation has completed, the fermented mash was centrifuged at 4000×g for 10 minutes to remove solids. The supernatant was filtered with a membrane, and ammonium sulfate was added. The precipitates from the fractions of 50-100% saturation were in turn dissolved with distilled water, dialysed and freeze-dried. Thus an antitumoral substance (4.8 gram) of light yellow powder was obtained.

Example 14

Pearl barley (1 kilogram) was ground, and mixed with water (2.7 liters), and commercially-available glucoamylase (2 gram), protease (1 gram), lipase (1 gram) and bakers yeast (3 gram) added thereto. For fermentation, the mixture was incubated at 28-30°C for 4 days.

To the fermented mash, acetic acid bacteria culture was inoculated and incubated at 28-32°C over 7 days for fermentation. When the last fermentation was completed, the fermented mash was centrifuged at 4000×g for 10 minutes to remove solids. The supernatant was filtered with a membrane to remove bacteria, and concentrated under reduced pressure. To the concentrate, was added ethanol, and precipitates produced in the ethanol concentration range of 20 to 80% were dissolved in a suitable amount of water, and freeze-dried. Thus an antitumoral substance (15.2 gram) of light gray powder was obtained.

Example 15

Unpolished rice (1 kilogram) was ground, and mixed with water (3.0 liters), and commercially-available glucoamylase (1 gram), α-amylase (0.5 gram), protease (1 gram), cellulase (0.5 gram) and precultured and collected fresh yeast (3 gram) added thereto. For fermentation, the mixture was incubated at 28-30°C for 6 days. To the fermented mash, acetic acid bacteria culture was inoculated and incubated at 28-30°C with aeration and stirring over 3 days for further fermentation. The fermentation liquor was concentrated by ultrafiltration, and then the addition of ethanol to it was made. Precipitates produced in the ethanol concentration range of 20 to 80% were dissolved in a suitable amount of water, and freeze-dried. Thus an antitumoral substance (16.9 gram) of light yellow powder was obtained.

Example 16

Corn (1 kilogram) was ground, and mixed with water (2.5 liters, and commercially-available glucoamylase (1 gram), protease (0.5 gram), pectinase (1 gram) and bakers yeast (3 gram) added thereto. For fermentation, the mixture was incubated at 28-30°C for 5 days. To the fermented mash, acetic acid bacteria culture was inoculated and incubated at 28-30°C over 6 days for stationary fermentation. This fermented mash was centrifuged at 4000×g for 10 minutes to remove solids. The supernatant was filtered with a membrane to remove bacteria, and concentrated in vacuo. To the concentrate, was added ethanol, and precipitates produced in the ethanol concentration range of 20 to 80% were dissolved in a suitable amount of water, and freeze-dried. Thus an antitumoral substance (5.3 gram) of light yellow powder was obtained.

Example 17

Pearl barley (1 kilogram) was ground, and mixed with water (3 liters), and commercially-available glucoamylase (2 gram), protease (1 gram) and bakers yeast (3 grams) added thereto. For dissolution, the mixture was incubated at 28-30°C for 12 days. the resultant mash was subjected to centrifugation (4000×g, 5 minutes) to remove solids. The supernatant was filtered with diatomaceous earth and a membrane (0.45μ m). To clear liquor, ammonium sulfate was added. The precipitates from the fractions of 50-100% saturation were in turn dissolved with distilled water, dialysed and freeze-dried. Thus an antitumoral substance (1.8 gram) of yellow powder was obtained.

Example 18

Pearl barley (1 kilogram) was ground, and mixed with water (2.7 liters), and commercially-available glucoamylase (2 gram), protease (1 gram), pectinase (0.5 gram) and previously cultured/collected fresh yeast (3 gram) added thereto. For dissolution, the mixture was incubated at 32-32°C for 14 days. The resultant mash was filtered to remove solids, and then subjected to ultrafiltration with a membrane (fractional MW: 10000), thereby 10 times higher concentration and desalting could be simultaneously accomplished. The concentrated liquor was freeze-dried. Thus an antitumoral substance (4.5 gram) of light yellowish powder was obtained.

Example 19

Pearl barley (1 kilogram) was ground, and mixed with water (3 liters), and commercially-available glucoamylase (2 gram), protease (1 gram), cellulase (0.5 gram) and bakers yeast (3 gram) added thereto. For dissolution, the mixture was incubated at 28-30°C for 14 days. the resultant mash was subjected to centrifugation (4000×g, 5 minutes) to remove solids. The supernatant was filtered with diatomaceous earth. To the filtrate, ammonium sulfate was added. The precipitates from the fractions of 50-100% saturation were in turn dissolved with distilled water, dialysed and freeze-dried. Thus an antitumoral substance (1.2 gram) of light yellow powder was obtained.

Example 20

Corn (1 kilogram) was ground, and mixed with water (2.7 liters), and commercially-available glucoamylase (2 gram), protease (2 gram), and bakers yeast (1 gram) added thereto. For dissolution, the mixture was incubated at 28-30°C for 14 days. The resultant mash was filtered with diatomaceous earth. To the filtrate, ammonium sulfate was added. The precipitates from the fractions of 50-100% saturation were in turn dissolved with distilled water, dialysed and freeze-dried. Thus an antitumoral substance (1.3 gram) of light yellow powder was obtained.

Example 21

Pearl barley (1 kilogram) was ground, mixed with water (2.7 liters), and adjusted with lactic acid to pH 4.5. To the mixture was added bakers yeast (1 gram) and previously-made preparation consisting of commercially-available glucoamylase (2 gram) dissolved in 20 milliliter of distilled water, and adjusted to pH 7.8, and treated at 40°C for 30 minutes to destroy the protease activity. For alcohol fermentation without cooking, the resultant mash was maintained at 28-30°C for 5 days.

To the fermented mash is added mother vinegar (1 liter) of acidity 8.0 to be denatured, and then filtration was made to separate into residue and vinegar mash. The latter was used for the production of vinegar. To the residue, water (4 liters) was added, and sterilized at 90°C for 5 minutes. After allowed to cool were added acetic acid (100 milliliter), commercially-available glucoamylase (2 gram), protease (2 gram) and pectinase (2 gram), and then extracted was made at 30°C for 7 days. Upon A.E. value has been

35%, centrifugation (4000ˣg, 5 minutes) was made to remove solids, followed by filtrations with diatomaceous earth and additionally a membrane (0.45μ m). To the clear liquor, ammonium sulfate was added. The precipitation fraction of 100% saturation were in turn dissolved with distilled water, dia lysed and freeze-dried. Thus an antitumoral substance (1.6 gram) of yellowish brown powder was obtained.

Example 22

To pearl barley (1 kilogram) were added water (2.7 liters) and commercially-available α-amylase (3 gram), and incubated at 60°C for 30 minutes for saccharification. Then the temperature of the mash was caused to rise to 120°C (2 atmospheric pressure) and maintained as it was for 5 minutes. Upon cooling to 55°C, adjustment with lactic acid to pH 5.0, followed by addition of commercially-available glucoamylase (3 gram), and thus saccharification was made for 16 hours.

The saccharified mash was filtered to remove solids, and the separated liquor was used as sugar solution. To the solid residue were added water (4 liters) acetic acid (100 milliliter), commercially-available glucoamylase (2 gram), protease (2 gram) and pectinase (2 gram), and then extraction was made at 30°C for 10 days. Upon reaching A.E. value 37%, filtration was made to remove solids. The filtration was processed in the same way as in Example 21 (but with regards to the supernatant). Thus an antitumoral substance (1.1 gram) of yellowish brown powder was obtained.

Example 23

Pearl barley (1 kilogram) were processed up to comple tion of saccharification in the same way as in Example 22, and adjustment with lactic acid to pH 4.5, followed by addition of commercially-available bakers yeast (3 gram), and thus alcohol fermentation was made at 28-30°C for 3 days. To the fermented mash, vinegar starter (acidity 8.0, 1 liter) was added to be denatured, and filtered to separate into solids and vinegar mash. The latter was used as for the production of vinegar. To the residue were added water (4 litters), acetic acid (100 milliliter), commercially-available glucoamylase (2 gram), protease (2 gram) and pectinase (2 gram), and then extracted was made at 30°C for 8 days. Upon reaching A.E. value 39%, centrifugation (4000ˣg, 5 minutes) was made to remove solids. The supernatant was processed in the same way as in Example 21. Thus an antitumoral substance (1.3 gram) of yellowish brown powder was obtained.

Example 24

With pearl barley (1 kilogram), proceeded up to completion of saccharification in the same way as in Example 22. After cooling, adjustment with lactic acid to pH 4.5, followed by addition of commercially-available bakers yeast (3 gram), glucoamylase (2 gram), protease (2 gram) and pectinase (2 gram), and thus alcohol fermentation was made at 28-30°C for 3 days. To the fermented liquor (6 liters) were added acetic acid (350 milliliter) and protease (2 gram), and incubated at 30°C for 12 days for extraction. Upon reaching A.E. value 42%, filtration was made to remove solids. The filtrate was processed in the same way as in the Example 21 (but with regards to the supernatant). Thus an antitumoral substance (1.2 gram) of yellowish brown powder was obtained.

Example 25

With corn (1 kilogram), proceeded up to completion of alcohol fermentation in the same way as in Example 24. To the fermented liquor (6 liters) were added acetic acid (350 milliliter), commercially-available protease (2 gram) and cellulase (2 gram), and incubated at 30°C for 17 days for extraction. Upon reaching A.E. value 46%, filtration was made to remove solids. The filtrate was processed in the same way as in Example 21 (but with regards to the supernatant). Thus an antitumoral substance (1.6 gram) of light brown powder was obtained.

18

## Claims

1. An antitumoral agent obtained in the process comprising extracting a proteinic substance from an unprocessed or processed seed having suffered thermal denaturation or having not, and purifying said proteinic substance.

2. An antitumoral agent defined in claim 1 wherein said proteinic substance comprising polypeptide of molecular weight 500-5000 is protein alone or a mixture essentially containing protein.

3. A process for preparing an antitumoral agent comprising extracting a proteinic substance from a mixture consisting of an unprocessed or processed seed having suffered no thermal denaturation and an aqueous medium added thereto, and subjecting said extracted proteinic substance to the action of enzyme; or subjecting said mixture to the action of enzyme and extracting a proteinic substance; and purifying said proteinic substance.

4. A process for preparing an antitumoral agent defined in claim 3 wherein said enzyme consists essentially of an endopeptidase or glucoamylase.

5. A process for preparing an antitumoral agent defined in claim 4 wherein purification of said proteinic substance is carried out after the rate of amino nitrogen to total nitrogen of said extract either having been subjected to the action of enzyme or extracted under the action of enzyme has been 10 to 70%.

6. A process for preparing an antitumoral agent comprising removing amylaceous substances from a seed having suffered no thermal denaturation by mechanical, enzymatic or biochemical means, or in combination thereof, extracting a proteinic substance with an aqueous medium alone or in combination with enzyme added thereto, and purifying proteinic substance from said extract containing said proteinic substance.

7. A process for preparing an antitumoral agent defined in claim 6 wherein said mechanical means comprises immersing in water, grinding and sieving said seed.

8. A process for preparing an antitumoral agent defined in claim 6 wherein said enzymatic means comprises subjecting a seed to the action of enzyme consisting essentially of glucoamylase.

9. A process for preparing an antitumoral agent defined in claim 6 wherein said enzyme consisting essentially of protease, glucoamylase and pectinase.

10. A process for preparing an antitumoral agent defined in claim 6 wherein said biochemical means comprises adding mould or yeast and causing to ferment.

11. A process for preparing an antitumoral agent comprising causing a seed having suffered no thermal denaturation to be fermentable; either adding enzyme alone to and leaching said altered seed, or adding enzyme in combination with yeast to and subjecting said altered seed to leaching/alcohol fermentation or additionally fermentation by the work of acetic acid bacterium inoculated; and causing the thus obtained fermentation liquor to be filtered; and purifying the filtered off proteinic substance.

12. A process for preparing an antitumoral agent defined in claim 11 wherein said enzyme consists essentially of glucoamylase, protease, $\alpha$-amylase and pectinase.

13. A process for preparing an antitumoral agent comprising grinding a seed having suffered no thermal denaturation, adding to the ground seed water of 2.0 to 7.0 times the amount of said ground seed, enzyme consisting essentially of glucoamylase and yeast, incubating the resulting mixture at 25-30°C for 3 to 6 days to accomplish alcohol fermentation, filtering or centrifuging the fermented mash to separate into fermentation liquor and residue, said fermentation liquor being used for acetic acid fermentation to produce vinegar, adding to the acetic acid fermentation residue an aqueous medium and enzyme consisting essentially of endopeptidase, subjecting the last mixture to extraction of proteinic substance, and purifying said proteinic substance, thus said antitumoral agent being obtained from said residue coming out of the vinegar producing process.

14. A process for preparing an antitumoral agent comprising subjecting an unprocessed or processed seed to heat-treatment, adding to the heat-treated seed an aqueous medium and enzyme, extracting proteinic substance from the mixture and purify proteinic substance from said extract.

15. A process for preparing an antitumoral agent defined in claim 14 wherein upon reaching amino acid equivalent (rate of amino nitrogen to total nitrogen; A.E.) 10 to 70%, said purification of proteinic substance being carried out.

16. A process for preparing an antitumoral agent comprising grinding a seed, adding to the ground seed water of 2.0 to 7.0 times the amount of the ground seed, subjecting the mixture to liquefaction of starch under the action of $\alpha$-amylase, cooking the resultant mash, adding to the cooked mash enzyme and yeast, incubating the second mixture at 25-35°C for 3 to 7 days to accomplish alcohol fermentation, denaturing the fementation liquor as predetermined, filtering or centrifuging the fermented mash to separate into fermentation liquor and residue, said fermentation liquor being used for acetic acid fermentation to produce vinegar, adding to the acetic acid fermentation residue an aqueous medium and enzyme, subjecting the last mixture to extraction of proteinic substance, and purifying said proteinic substance, thus said antitumoral agent being obtained from said residue coming out of the vinegar producing process.

17. A process for preparing an antitumoral agent comprising grinding a seed, adding to the ground seed water of 2.0 to 7.0 times the amount of the ground seed, subjecting the mixture to liquefaction of starch under the action of α-amylase and to saccharification under the action of glucoamylase, subjecting the resultant mash to, after cooking filtering or centrifuging the saccharified mash to separate into fermentation liquor and residue, said fermentation liquor being used as sugar solution or for alcohol fermentation, adding to said residue an aqueous medium and enzyme, subjecting the last mixture to extraction of proteinic substance, and purifying said proteinic substance, thus said antitumoral agent being obtained from said residue coming out of the sugar solution producing process.